# EUROPEAN PATENT SPECIFICATION

(11) **EP 2 181 645 B1**
(45) Date of publication and mention of the grant of the patent: **03.09.2014**
(21) Application number: 10150239.1
(22) Date of filing: 20.04.2006
(51) Int. Cl.: A61B 1/00, A61B 1/313, A61B 19/00, A61B 8/00, A61B 17/34, A61B 17/00

(54) **Device of improving laparoscopic surgery**
Vorrichtung zur Verbesserung der Laparoskopie-Chirurgie
Dispositif d'amélioration de chirurgie de laparoscopie

(30) Priority: 18.04.2005 US 672010 P; 04.08.2005 US 705199 P; 15.09.2005 US 716951 P; 15.09.2005 US 716953 P
(43) Date of publication of application: 05.05.2010
(62) Divisional of application: 06728279.8
(73) Proprietor: M.S.T. Medical Surgery Technologies Ltd, Nazereth (IL)
(72) Inventor: Sholev, Mordehai, 37830, Amikam (IL)
(74) Representative: Lecomte & Partners

(56) References cited:
- US-A- 5 279 309
- US-A- 5 749 362
- US-A1- 2002 156 466
- US-A1- 2004 015 053
- US-A1- 2004 204 627
- US-B1- 6 714 841

## Description

### FIELD OF THE INVENTION

The present invention relates to means and methods for improving the interface between the surgeon and an endoscope system for laparoscopic surgery. Moreover, this present invention discloses a device useful for controlling an endoscope system for laparoscopic surgery, in which the endoscope is inserted through a small incision.

### BACKGROUND OF THE INVENTION

In laparoscopic surgery, the surgeon performs the operation through small holes using long instruments and observing the internal anatomy with an endoscope camera. The endoscope is conventionally held by a camera assistant since the surgeon must perform the operation using both hands. The surgeon performance is largely dependent on the camera position relative to the instruments and on a stable image shown at the monitor. To overcome these problems, several new technologies have been developed, using robots to hold the endoscope while the surgeon performs the procedure, e.g., Lapman, Endoassist etc. But these technologies are expensive, difficultly installed, uncomfortable to use, limiting the dexterity of the surgeon and having physical dimension much bigger that all operating tools. Relatively to the required action, they also move in big bounds with several arms movement.

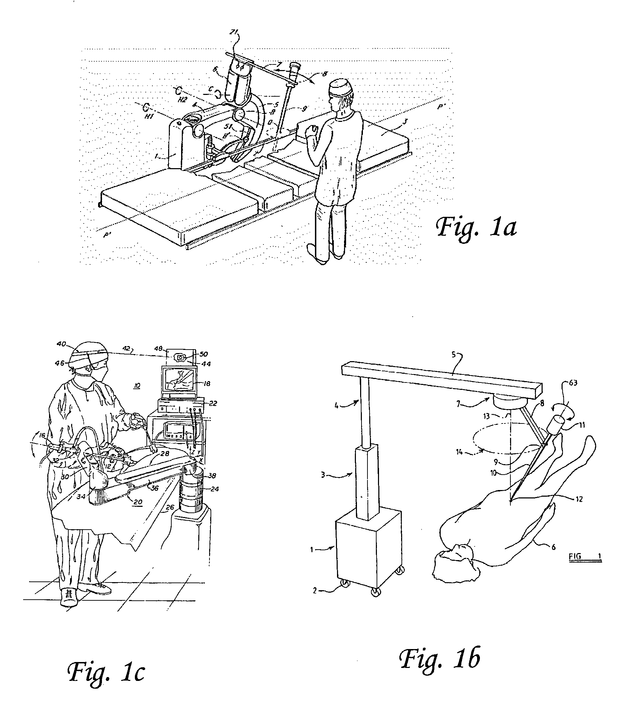

Reference is made now to figures 1a, 1b 1c, presenting a schematic illustration of the prior art which describes these technologies.

Laparoscopic surgery is becoming increasingly popular with patients because the scars are smaller and their period of recovery is shorter. Laparoscopic surgery requires special training of the surgeon or gynecologist and the theatre nursing staff. The equipment is often expensive and not available in all hospitals. During laparoscopic surgery it is often required to shift the spatial placement of the endoscope in order to present the surgeon with the optimal view. Conventional laparoscopic surgery makes use of either human assistants that manually shift the instrumentation or alternatively robotic automated assistants.

Automated assistants utilize interfaces that enable the surgeon to direct the mechanical movement of the assistant, achieving a shift in the camera view. US patent no. 5279309 relates to a system and method that includes a manipulator for manipulating a surgical instrument relative to a patient's body and, a position sensor for sensing the position of the surgical instrument relative to the patient's body. The manipulator can be manually or computer actuated and can have brakes to limit movement. In a preferred embodiment, of US patent no. 5279309 orthogonal only motion between members of the manipulator is provided. The position sensor includes beacons connected to the patient and manipulator or surgical instrument and, a three dimensional beacon sensor adapted to sense the location and position of the beacons. Redundant joint sensors on the manipulator may also be provided. The system and method uses a computer to actively interact with the surgeon and can use various different input and output devices and modes.

US patent 6,714,841 discloses an automated camera endoscope in which the surgeon is fitted with a head mounted light source that transmits the head movements to a sensor, forming an interface that converts the movements to directions for the mechanical movement of the automated assistant. Alternative automated assistants incorporate a voice operated interface, a directional key interface, or other navigational interfaces. The above interfaces share the following drawbacks:
a. Single directional interface that provides limited feedback to the surgeon.
b. Cumbersome serial operation for starting and stopping movement directions that requires the surgeon's constant attention.

Research has suggested that these systems divert the surgeons focus from the major task at hand. Therefore technologies assisted by magnets and image processing have been developed to simplify interfacing control. However these improved technologies still fail to address another complicating interface aspect of laparoscopic surgery, they do not allow the surgeon to signal to both the automated assistant and to surgical colleagues, which instrument his attention is focused on.

### SUMMARY OF THE INVENTION

It is one object of the present invention to disclose a device as defined in claim 1.

It is still in the scope of the present invention wherein each surgical device is fitted with a wireless transmitter.

It is still in the scope of the present invention wherein said wireless transmitter is freestanding.

It is another object of the present invention to disclose the device as defined above, wherein said selection of said desired instrument is confirmed by clicking on said at least one key.

It is another object of the present invention to disclose the device as defined above, wherein said selection of said desired instrument is confirmed by depression of said at least one key on said wireless transmitter.

It is another object of the present invention to disclose the device as defined above, wherein said depression of said at least one key is a prolonged depression.

### BRIEF DESCRIPTION OF THE FIGURES

In order to understand the invention and to see how it may be implemented in practice, and by way of non-limiting example only, with reference to the accompanying drawing, in which
FIG. 1a, 1b 1c present a schematic illustration of prior art technologies;
FIG. 2 is a general schematic view of an enhanced interface laparoscopic system that relies on a single wireless code signal to indicate the instrument on which to focus the endoscope constructed in accordance with the principles of the present invention in a preferred embodiment thereof;
FIG. 3 is a general schematic view of an enhanced interface laparoscopic system that relies on at least two wireless signals to indicate the instrument on which to focus the endoscope;
FIG. 4 is a schematic view of the method in which the single wireless code signal choice instrumentation focus is represented on the viewing apparatus;
FIG. 5 is a schematic view of the method in which multiple wireless code signal choice of instrumentation is operated;

### DETAILED DESCRIPTION OF THE EMBODIMENTS

The following description is provided, alongside all chapters of the present invention, so as to enable any person skilled in the art to make use of the invention and sets forth the best modes contemplated by the inventor of carrying out this invention. Various modifications, however, will remain apparent to those skilled in the art, since the generic principles of the present invention have been defined specifically to provide means and methods for improving the interface between the surgeon and an endoscope system for laparoscopic surgery.

It is another object of the present invention to present a novel means for controlling the spatial position of endoscope tube in laparoscopic surgery. The present device is cheap, easily install and disassemble, comfortable to use, not limiting the dexterity of the surgeon and having small physical dimension.

The small size of present invention is achieved by applying the following steps:
1. separating the moving parts from the motors and transmitting the motor power by cable means;
2. applying a linear zoom mechanism, allowing a full range zoom action, independent of other moving parts in the mechanism, e.g. not like other robots that achieve the linear zoom action, by a combined movement of the robot arms;
3. obtaining a rotational mechanism that rotates the endoscope about its long axis, independently of other moving parts of the mechanism, e.g., not like other robots that does not have the ability to compensate un wanted rotational movements, or by a combined movement of the robot arms that produce big movements in order to achieve small rotations.

As mention, the present invention can be also utilized to improve the interface between the surgeon and the endoscope system. Moreover, the present invention can be also utilized to control and/or direct an automated endoscope assistant to focus the endoscope to the desired instrument of the surgeon. Furthermore, the device is adapted to focus the camera view on the selected instrument area.

In preferred embodiment of the invention a single wireless emission code is utilized and choice is achieved by a visible graphic representation upon the conventional viewing screen.

In another preferred embodiment each instrument is fitted with a unique code wireless transmitter, and selection is achieved by depressing its button.

The present invention discloses also a device joined with conventional camera assisted laparoscopic surgery systems comprising at least one wireless transmitter that may or may not be attached to the maneuvering control end of surgical instruments.

Upon depression of at least one button on the transmitters either a generic or a unique code is transmitted to a receiving device connected to a computer that presents (e.g. displays) the selected surgical tool on a connected video screen. Confirmation of the selection by the depression of at least one button on wireless transmitter transmits a code to the receiver connected to the computer that instructs the automated surgical assistant to move the endoscope achieving a view on the screen that is focused on the selected instrument area.

It would thus be desirable to achieve a device that allows the surgeon to identify to the laparoscopic computing system as well as to surgical colleagues to which surgical instrument attention is to be directed_thereby directing the view achieved by the endoscope to the selected focus of attention.

Therefore, in accordance with a preferred embodiment of the present invention an enhanced interface laparoscopy device is provided. The device comprising:
a. At least one wireless transmitter with at least one operating key.
b. At least one wireless receiver.
c. at least one conventional laparoscopy computerized system; said conventional laparoscopy computerized system loaded with conventional_surgical instrument spatial locating software, and a conventional automated assistant maneuvering software; said locating software enables a visual response to the depression of said at least one key on said wireless transmitter; said maneuvering software achieves the movement of said endoscope.
d. At least one video screen.
e. At least one automated assistant.

In a preferred embodiment of the enhanced interface laparoscopy device the wireless transmitter or transmitters are either freestanding or attached to the maneuvering end of the surgical instruments and emit the same single code that upon the depression of at least one key on them emits a signal to the receiver that communicates with the connected computer that displays a graphic symbol upon a random choice of one of the onscreen surgical instruments depicted by the computer on the screen. The surgeon repeats the depression of at least one key resulting in a shift in the displayed graphic designator from one onscreen depiction of surgical instrument to another until the desired instrument is reached and thereby selected. Subsequently the computer directs the automated assistant to focus the endoscope on the desired instrument area.

In a further preferred embodiment the selection of the instrument requires confirmation by varying the form of click on at least one key, such as a prolonged depression. Only upon confirmation is the computer authorized to instruct the automated assistant to focus the endoscope on the desired instrument area.

In another preferred embodiment of the invention each relevant surgical instruments is fitted at its maneuvering control end with a wireless transmitter with at least one key that transmits a unique code. In the initial stage of the procedure the surgeon identifies each of the instruments to the computerized system by depressing at least one key on each of the wireless transmitters fitted to the surgical instruments and matching their characteristics with a prepared database, thereby forming within the computerized system a unique signature for each of the transmitters. Thereon, upon depression of at least one key on the wireless transmitter attached to each surgical instrument, the receiver receives the unique code communicates it to the computer that identifies it with the preprogrammed signature and instructs the automated assistant to move the endoscope so as to achieve the desired focus.

In another preferred embodiment of the invention each relevant surgical instruments is fitted at its maneuvering control end with a wireless transmitter with at least one key that transmits a unique code. In the initial stage of the procedure the surgeon identifies each of the instruments to the computerized system by depressing at least one key on each of the wireless transmitters fitted to the surgical instruments and matching their characteristics with a prepared database, thereby forming within the computerized system a unique signature for each of the transmitters. Thereon, upon depression of at least one key on the wireless transmitter attached to each surgical instrument, the receiver receives the unique code, communicates it to the computer that identifies it with the preprogrammed signature stored and instructs the automated assistant to move the endoscope so as to achieve the desired focus.

In a further preferred embodiment the selection is signified on the connected screen by displaying a graphic symbol upon the onscreen depiction of the surgical instrument.

In a further preferred embodiment the selection is confirmed by an additional mode of depression of at least one key on the wireless transmitter, such as a prolonged depression of the key, authorizing the computer to instruct the automated assistant to change view provided by the endoscope.

The device of the present invention has many technological advantages, among them:
- Simplifying the communication interface between surgeon and mechanical assistants.
- Seamless interaction with conventional computerized automated endoscope systems.
- Simplicity of construction and reliability.
- User-friendliness
- Additional features and advantages of the invention will become apparent from the following drawings and description.

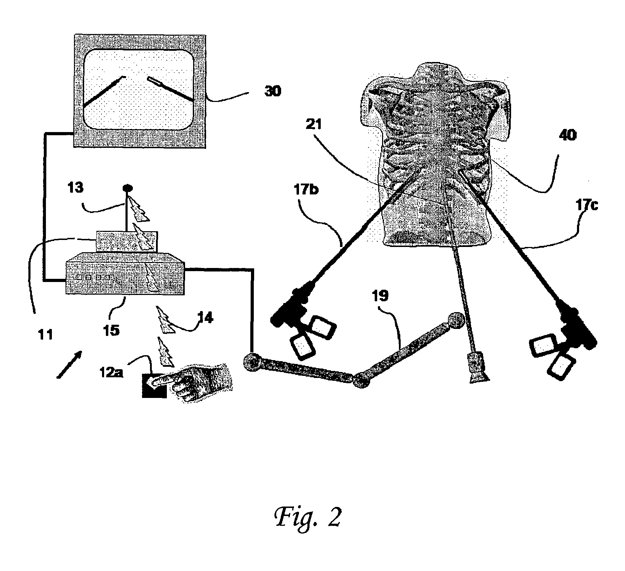

Reference is made now to figure 2, which is a general schematic view of an enhanced interface laparoscopic system comprising one or more button operated wireless transmitters 12a, that may or may not be attached to the maneuvering end of surgical instruments 17b and 17c, which once depressed aerially transmit a single code wave 14 through aerial 13 to connected receiver 11 that produces a signal processed by computer 15 thereby assigning a particular one of two or more surgical instruments 17b and 17c as the focus of the surgeons attention. Accordingly a conventional automated endoscope 21 is maneuvered by means of conventional automated arm 19 according to conventional computational spatial placement software contained in computer 15.

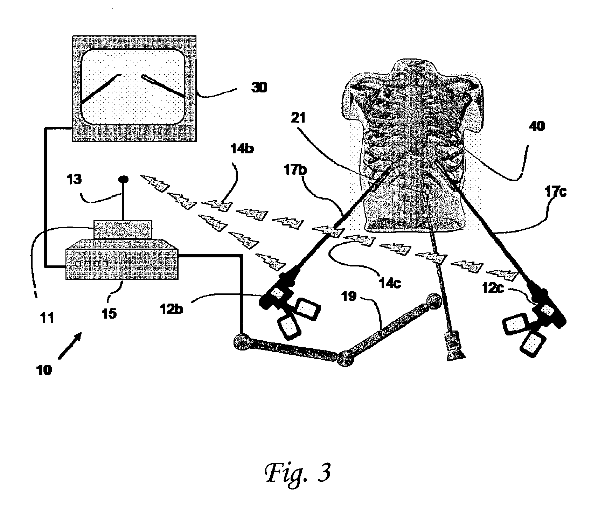

Reference is made now to figure 3, which is a general schematic view of an enhanced interface laparoscopic system comprising one or more button operated wireless transmitters 12b and 12c are attached respectfully to the maneuvering means at the end of surgical instruments 17b and 17c, which once depressed aerially, each transmit a unique code wave 14b and 14c through aerial 13 to connected receiver 11 that produces a signal processed by computer 15 thereby assigning a particular one of two or more surgical instruments 17b and 17c as the focus of the surgeons attention. Accordingly a conventional automated endoscope 21 is maneuvered by means of conventional automated arm 19 according to conventional computational spatial placement software contained in computer 15.

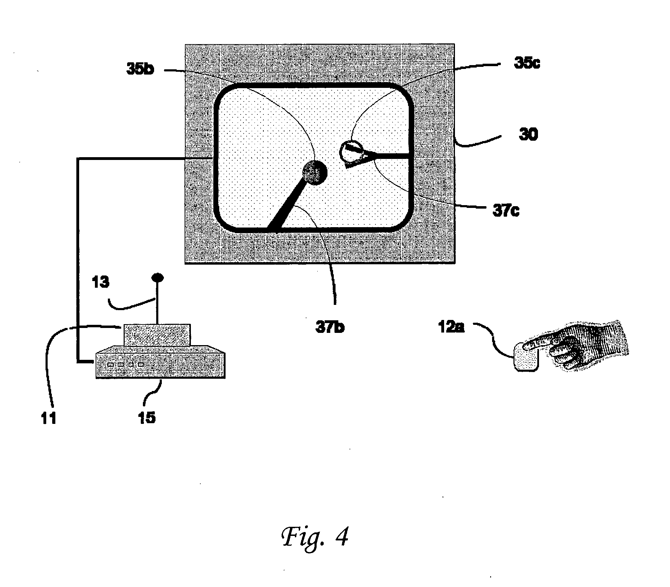

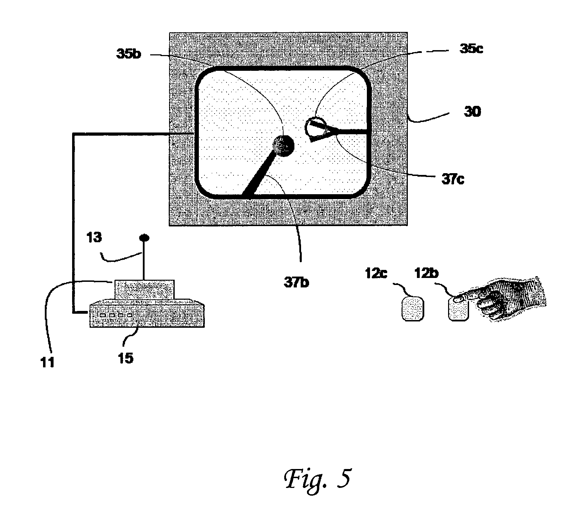

Reference is made now to figure 4, which is a schematic view of the method in which single wireless signal code choice of instrumentation focus is achieved, by means of video representation, 35b and 35c of the actual surgical instruments (not represented in fig. 4) displayed by graphic symbols. Wherein a light depression of the button on generic code emitting wireless transmitter 12a transmits a code that is received by receiver aerial 13 communicated through connected receiver 11 to computer 15 that shifts the graphically displayed symbol of choice 35b on video screen 30 from instrument to instrument until the required instrument is reached. A prolonged depression of the button on transmitter 12a confirms the selection thereby signaling computer 15 to instruct the automated mechanical assistant (not represented in fig. 5) to move the endoscope (not represented in fig. 4) and achieving a camera view of the instrument area on screen 30.

Reference is made now to figure 5, which is a schematic view of the method in which multiple wireless signal code choice of instrumentation focus is achieved, by means of video representation 35b and 35c of the actual surgical instruments (not represented in fig. 5) displayed by graphic symbols. Wherein when buttons on unique code emitting wireless transmitters 12b and 12c attached respectfully to actual operational instruments (not represented in fig. 5) displays graphic symbol 35b on respectful video representation 37b. A prolonged depression of the button on transmitter 12b and 12c confirms the selection thereby signaling computer 15 to instruct the automated mechanical assistant (not represented in fig. 5) to move the endoscope (not represented in fig. 5) and achieving a camera view of the instrument area on screen 30.

In order to realize a position and range system, many well known technologies may be used. For example if the switches emit wireless signals then an array of antennas may be used to compare the power of the signal received at each antenna in order to determine the angle of the switch and it's approximate range to the camera holder mechanism. If the switch emits ultra sound wave then US microphones can be used to triangulate the position of the switch. The same is for light emitting switch.

## Claims

1. A device useful for the interface between a surgeon and an automated assistant, comprising:
a. an endoscope (21), mechanically interconnected to said automated assistant (19);
b. at least one surgical instrument (17b, 17c);
c. at least one wireless transmitter (12a, 12b, 12c) with at least one operating key; said wireless transmitter (12a, 12b, 12c) and said at least one operating key are attached to the maneuvering end of said at least one surgical instrument (17b, 17c); said wireless transmitter is adapted to transmit a signal (14) once said at least one operating key is pressed;
d. at least one wireless receiver (11); adapted to receive said signal (14) sent by said transmitter (12a, 12b, 12c);
e. at least one conventional laparoscopy computerized system, in communication with said wireless receiver, said conventional laparoscopy computerized system is loaded with (i) conventional surgical instrument spatial location software; (ii) conventional automated assistant maneuvering software being in communication with said automated assistant adapted to maneuver said endoscope; said conventional laparoscopy computerized system is loaded with software adapted to provide a visual onscreen depiction of said at least one surgical instrument (17b, 17c) to be selected following a depression of said at least one operating key; and,
f. at least one video screen (30);
said device is adapted to control and to direct said endoscope (21) via said automated assistant on said instrument of said surgeon.

2. The device according to claim 1, wherein said wireless transmitter (12a, 12b, 12c) is freestanding.

3. The device according to claim 1, wherein each of said at least instruments (17b, 17c) is fitted with at least one of said wireless transmitters (12a, 12b, 12c).

4. The device according to claim 3, wherein said wireless transmitter (12a, 12b, 12c) is adapted to locate the position of at least of said instruments (17b, 17c).

5. The device according to claim 1, wherein a selection of said at least one instrument is confirmable by clicking on said at least one operating key.

6. The device according to claim 1, wherein a selection of said desired instrument is confirmable by depression of said at least one key on said wireless transmitter (12a, 12b, 12c).

7. The device according to claim 6, wherein said depression of said at least one key is a prolonged depression.

## Patentansprüche

1. Vorrichtung, welche nützlich für die Schnittstelle zwischen einem Chirurgen und einem automatisierten Assistenten ist, umfassend:
a. ein Endoskop (21), das mechanisch mit dem automatisierten Assistenten (19) verbunden ist;
b. mindestens ein chirurgisches Instrument (17b, 17c);
c. mindestens einen drahtlosen Sender (12a, 12b, 12c) mit mindestens einer Bedientaste; der drahtlose Sender (12a, 12b, 12c) und die mindestens eine Bedientaste sind an dem Manövrierende des mindestens einen chirurgischen Instruments (17b, 17c) befestigt; der drahtlose Sender ist dazu eingerichtet, ein Signal (14) zu senden, sobald die mindestens eine Bedientaste gedrückt wird;
d. mindestens einen drahtlosen Empfänger (11); dieser ist dazu eingerichtet, das von dem Sender (12a, 12b, 12c) gesendete Signal (14) zu empfangen;
e. mindestens ein herkömmliches computergestütztes Laparoskopiesystem in Kommunikation mit dem drahtlosen Empfänger, wobei das herkömmliche computergestützte Laparoskopiesystem versehen ist mit (i) herkömmlicher Software zur räumlichen Ortung chirurgischer Instrumente; (ii) herkömmlicher Software zum Manövrieren von automatisierten Assistenten, die in Kommunikation mit dem automatisierten Assistenten steht, der zum Manövrieren des Endoskops eingerichtet ist; wobei das herkömmliche computergestützte Laparoskopiesystem mit Software versehen ist, die dazu eingerichtet ist, eine visuelle Bildschirmdarstellung des mindestens einen chirurgischen Instruments (17b, 17c) vorzusehen, das nach Drücken der mindestens einen Bedientaste auszuwählen ist; und
f. mindestens einen Videobildschirm (30);
wobei die Vorrichtung zur Steuerung und Lenkung des Endoskops (21) mittels des automatisierten Assistenten an dem Instrument des Chirurgen eingerichtet ist.

2. Vorrichtung nach Anspruch 1, wobei der drahtlose Sender (12a, 12b, 12c) freistehend ist.

3. Vorrichtung nach Anspruch 1, wobei jedes der mindestens einen Instrumente (17b, 17c) mit mindestens einem der drahtlosen Sender (12a, 12b, 12c) ausgestattet ist.

4. Vorrichtung nach Anspruch 3, wobei der drahtlose Sender (12a, 12b, 12c) zur Ortung der Position von mindestens einem der Instrumente (17b, 17c) eingerichtet ist.

5. Vorrichtung nach Anspruch 1, wobei eine Auswahl des mindestens einen Instruments durch Anklicken der mindestens einen Bedientaste bestätigbar ist.

6. Vorrichtung nach Anspruch 1, wobei eine Auswahl des gewünschten Instruments durch Drücken der mindestens einen Taste an dem drahtlosen Sender (12a, 12b, 12c) bestätigbar ist.

7. Vorrichtung nach Anspruch 6, wobei das Drücken der mindestens einen Taste ein längeres Drücken ist.

## Revendications

1. Dispositif utile pour l'interface entre un chirurgien et un assistant automatisé, comprenant :
a. un endoscope (21) interconnecté par voie mécanique audit assistant automatisé (19) ;
b. au moins un instrument chirurgical (17b, 17c) ;
c. au moins un émetteur sans fil (12a, 12b, 12c) comprenant au moins une touche d'utilisation, ledit émetteur sans fil (12a, 12b, 12c) et ladite au moins une touche d'utilisation étant fixés à l'extrémité de manoeuvre dudit au moins un instrument chirurgical (17b, 17c), ledit émetteur sans fil étant conçu pour émettre un signal (14) une fois que l'on appuie sur ladite au moins une touche d'utilisation ;
d. au moins un récepteur sans fil (11) conçu pour recevoir ledit signal (14) envoyé par ledit émetteur sans fil (12a, 12b, 12c) ;
e. au moins un système informatisé conventionnel de laparoscopie en communication avec ledit récepteur sans fil, ledit système informatisé conventionnel de laparoscopie étant chargé avec (i) un logiciel conventionnel de localisation spatiale d'un instrument chirurgical, (ii) un logiciel conventionnel de manoeuvre de l'assistant automatisé mis en communication avec ledit assistant automatisé, conçu pour manoeuvrer ledit endoscope, ledit système informatisé conventionnel de laparoscopie étant chargé avec un logiciel conçu pour procurer une représentation visuelle sur écran dudit au moins un instrument chirurgical (17b, 17c) à sélectionner après avoir appuyé sur ladite au moins une touche d'utilisation ; et
f. au moins un écran vidéo (30) ;
ledit dispositif étant conçu pour commander et pour diriger ledit endoscope (21) via ledit assistant automatisé sur ledit instrument dudit chirurgien.

2. Dispositif selon la revendication 1, dans lequel ledit émetteur sans fil (12a, 12b, 12c) est autonome.

3. Dispositif selon la revendication 1, dans lequel chacun desdits au moins un instrument chirurgical (17b, 17c) est équipé d'au moins un desdits émetteurs sans fil (12a, 12b, 12c).

4. Dispositif selon la revendication 3, dans lequel ledit émetteur sans fil (12a, 12b, 12c) est conçu pour localiser la position desdits au moins un instrument chirurgical (17b, 17c).

5. Dispositif selon la revendication 1, dans lequel une sélection desdits au moins un instrument peut être confirmée en cliquant sur ladite au moins une touche d'utilisation.

6. Dispositif selon la revendication 1, dans lequel une sélection desdits au moins un instrument peut être confirmée en appuyant sur ladite au moins une touche sur ledit émetteur sans fil (12a, 12b, 12c).

7. Dispositif selon la revendication 6, dans lequel ledit appui sur ladite au moins une touche est un appui prolongé.
